(19) Europäisches Patentamt
European Patent Office
Office européen des brevets

(11) Publication number: **0 116 784 B1**

# (12) EUROPEAN PATENT SPECIFICATION

(45) Date of publication of patent specification: **10.06.92**

(51) Int. Cl.5: **A61K 37/02**, A61K 35/34, C07K 7/10

(21) Application number: **83307877.7**

(22) Date of filing: **22.12.83**

The file contains technical information submitted after the application was filed and not included in this specification

## (54) Atrial natriuretic factor.

(30) Priority: **21.02.83 CA 421998**
**09.03.83 US 473442**
**31.10.83 US 546817**

(43) Date of publication of application:
**29.08.84 Bulletin 84/35**

(45) Publication of the grant of the patent:
**10.06.92 Bulletin 92/24**

(84) Designated Contracting States:
**BE CH DE FR GB IT LI NL SE**

(56) References cited:

**CHEMICAL ABSTRACTS, vol.97, 1982, Columbus, OH (US); A.J.DE BOLD, p.127, no.66940r**

**CHEMICAL ABSTRACTS, vol.99, 1983, Columbus, OH (US); A.J.DE BOLD et al., p.91, no.99484q**

**BIOLOGICAL ABSTRACTS, vol.76, 1983, Biological AbstractsInc., Philadelphia, PA (US); A.J.DE BOLD et al., no.51709**

**CHEMICAL ABSTRACTS, vol.94, 1981, Columbus, OH (US); A.J.DE BOLD et al., p.494, no.62919f**

**PROCEEDINGS OF THE SOCIETY FOR EXPERIMENTAL BIOLOGY & MEDICINE, vol.170, no.2, 1982; pp.133-138**

(73) Proprietor: **OUEEN'S UNIVERSITY AT KINGSTON**

**Kingston Ontario K7L 3N6(CA)**

(72) Inventor: **De Bold, Adolfo Jose**
**981 Alpine Place**
**Kingston, ON, K7P 1N8(CA)**

(74) Representative: **Brock, Peter William et al**
**UROUHART-DYKES & LORD 91 Wimpole Street**
**London W1M 8AH(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 116 784 B1

## Description

This invention relates to a diuretic and natriuretic peptide composition extracted from mammalian heart atria, especially rat hearts.

It is known that the muscle cells of the atrial myocardium in mammals contain, in addition to contractile elements similar to those found in ventricular fibers, a highly developed Golgi complex, a relatively high proportion of rough endoplasmic reticulum, and numerous membrane bound storage granules, referred to as specific atrial granules. No such granules appear to exist in ventricular muscle cells. Morphologically and histochemically the atrial granules resemble those present in polypeptide-hormone producing cells (J. Histochem. Cytochem. 26, 1094 - 1102 (1978) de Bold et al). It is also known (Life Sciences, Vol. 28 pp 89-94 (1981) de Bold et al) that injection of a crude extract of rat atrial myocardium induces a very potent and immediate natriuretic response in non-diuretic assay rats. The problem remains, however, to isolate and identify the atrial natriuretic factor (ANF) contained in the crude extract of the specific atrial granules, and which is hereinafter referred to as cardionatrin I.

A further paper by A.J.de Bold, in Proceedings of the Society for Experimental Biology and Medicine, 170, 133-138 (1982) discloses the partial purification of an extract from rat heart atria. An acetic acid extract from the atria was partially purified by chromatography and the active natriuretic factor appeared to be a peptide having a molecular weight below 6000.

In the course of further studies it has now been possible to isolate and determine the structure of an active peptide from rat heart atria.

This invention provides a peptide composition having diuretic and natriuretic properties, characterized by an amino acid sequence comprising:

Serine - Leucine - Arginine - Arginine - Serine -
Serine - Cysteine - Phenylalanine - Glycine -
Glycine - Arginine - Isoleucine - Aspartic acid -
Arginine - Isoleucine - Glycine - Alanine -
Glutamine - Serine - Glycine - Leucine -
Glycine - Cysteine - Asparagine - Serine -
Phenylalanine- Arginine - Tyrosine.

This invention also provides a polypeptide having the sequence Cysteine - Asparagine - Serine - phenylalanine - Arginine - Tyrosine at the C-terminal thereof, and G disulfide bond between the said cysteine residue and an other cysteine residue in the polypeptide.

This invention further provides a substantially pure polypeptide having hypotensive, diuretic and natriuretic properties, said diuretic and natriuretic properties enabling the polypeptide to increase the normal diuretic and natriuretic excretions by at least a two fold increase in urine output and a four fold increase in sodium excretion on injection of 0.5 nmol of said polypeptide into a non-diuretic rat, said polypeptide being free of tryptophan residues and containing cysteine residues which are bridged by a disulfide double bond.

The described work related to the extraction and characterization of ANF (cardionatrin I ) has only an illustrative value and is not an object of the present invention.

Figure 1 is a graph illustrating chromatographic desalting of rat atrial extract in Bio-Gel® P-2. Sample size: 10 ml of acetic acid extract obtained from 5 g of tissue. Column size: 2.6 x 60 cm. Fraction Size: 11 ml. Eluant: 1.0 M acetic acid. Linear flow rate: 2 cm/h. Hatched area indicates fractions where natriuretic activity is recovered;

Figure 2 is a graph illustrating chromatography of rat atrial extract (after desalting in Bio-Gel® P-2) in Sephadex® G-75. Column size 2.6 x 90 cm. Fraction size 11 ml. Eluant: 1.0 M acetic acid. Linear flow rate: 5 cm/h. Natriuretic activity (bars) was determined by injecting freeze-dried aliquots of pooled fractions resuspended in PBS;

Figure 3 is a bar graph illustrating typical effect of an injection of partially purified rat atrial natriuretic factor on arterial blood pressure and urine output (in drops) of bioassay rats;

Figure 4 is a graph illustrating net natriuretic and diuretic effect of partially purified natriuretic factor injected in a total volume of 0.2 ml of PBS. Net values were obtained by subtracting total sodium excretions and urine output of animals injected with PBS alone from corresponding values of animals

injected with natriuretic factor. (Values ± S.E.M.); and

Figure 5 is a bar graph illustrating ion exchange chromatography purification of partially purified natriuretic factor. Hatched area indicates fractions where natriuretic activity is recovered.

Figure 6 is an RP-HPLC elution profile of an acid extract of rat atrial muscle.

Figure 7 is an RP-HPLC elution profile of region I of Figure 6, after chromatography in HFBA-containing $CH_3CN$ gradient;

Figures 8a and 8b are RP-HPLC elution profiles obtained after rechromatography in a TFA-containing $CH_3CN$ gradient of active material from chromatography using HFBA (Figure 7);

Figure 9 is a graph of molecular weight versus migrated distance for SDS-Page comparison; and

Figure 10 is a UV absorption spectrum of cardionatrin I.

## DETAILED DESCRIPTION OF PREFERRED EMBODIMENTS

The presence of secretory-like specific atrial granules in cardiac atrial muscle fibers which has been recognized for some years (J. Cell. Biol. 23, 151 (1964) Jamieson et al) and the more recent finding that crude rat heart atrial muscle extracts are able to induce a very potent diuretic and natriuretic response in assay rats (Life Sciences 28, 89, (1981) de Bold et al) is believed to be of considerable importance to studies for in-vivo water and electrolyte balance. The problem remains, however, that the crude extract heretofore produced is not sufficiently purified to make a positive identification of the active factor. While a simple homogenization of heart tissue in water or buffered solutions may be employed for extraction of ANF, such a procedure is relatively slow and the concentration of the resulting ANF is very low. Upgrading is tedious and costly. Somewhat surprisingly, although extractions from large mammalian hearts, such as beef, can be effected the natriuretic factor therein appears to be but a very minor component even in the active U.V.-absorbing material isolated after ion exchange chromatography and the total extractable activity appears to be much lower than that obtained from an equivalent amount of rat tissue. Rat atria are therefore the preferred source material.

It has now been determined that advantage may be taken of the well known peptide-solvent properties of lower carboxylic acids such as formic and acetic acid etc., and two alternative techniques will be described in more detail hereinafter. In both techniques, rat atria provided the source material. In the first technique use is made of the extractive properties of 1.0 M acetic or other lower carboxylic acid optionally containing protease inhibitors. Following extraction, some contaminants are elminated by precipitation after adjusting the pH to a range between about 4.5 to about 7.6. The volume of the extract is then reduced, preferably by freeze drying and further purified by gel chromatography and ion exchange chromatography.

### Example 1

Atria were obtained from male Sprague-Dawley rats (300-350 g). Up to 100 atria (10 g wet weight) were used in each experiment. The animals had free access to food and water until sacrificed by decapitation. The hearts were rapidly removed and placed in ice cold phosphate buffered saline (PBS: 0.9% NaCl in 5 mM sodium phosphate buffer, pH 7.2). The atria were dissected, thoroughly rinsed in PBS, blotted dry and weighed. Ventricular tissues were similarly treated to provide controls. Five volumes of cold 1.0 M acetic acid, containing 1 mg of the protease inhibitors pepstatin A and phenylmethylsulfomyl fluoride, were added per gram of tissue wet weight. The tissue was then homogenized in a Polytron®, let to stand for 1 h on ice and centrifuged at 4° C for 30 min at 10,000 g. The supernatant from this centrifugation was saved and the pellet re-homogenized in 2.5 volumes of acetic acid and treated as above. The combined supernatant from the two acetic acid extractions was then adjusted to pH 7.6 and centrifuged once again. The supernatant from this centrifugation was freeze-dried, resuspended in 1.0 M acetic acid and desalted at 4° C in a Bio-Gel® P-2 column (2.6 x 60 cm) equilibrated with 1.0 M acetic acid. The void volume from this column was freeze-dried, resuspended in 1.0 M acetic acid and applied to a Sephadex® G-75 column (2.6 x 90 cm) equilibrated with 1.0 M acetic acid. Pooled active fractions obtained after this chromatography were freeze-dried. The product obtained is referred to as "partially purified natriuretic factor".

### Example 2

To study the effect of protease (Pronase) on partially purified natriuretic factor, dilutions were made in 0.05 M TRIS buffer, pH 7.6. Digestions were carried out by incubating 50 µg of partially purified natriuretic factor with 0.1 U of Pronase for 4 h at 37° C in a total volume of 1.1 ml of 0.05 M TRIS buffer, pH 7.6. The reaction was terminated by addition of 0.2 ml of glacial acetic acid and cooled on ice. After freeze-drying,

the samples were resuspended in 1.0 ml of PBS, centrifuged, and 0.2 ml aliquots tested for natriuretic activity.

Further purification of natriuretic factor was accomplished by ion exchange chromatography in CM Bio-Gel® equilibrated with 0.1 M ammonium acetate buffer, pH 4.7. Column size was 0.9 x 12 cm. Elution was carried out with 13 column volumes under starting conditions followed by a 13 column volumes gradient to 0.1 M ammonium acetate, pH 7.0 and, finally, with 13 column volumes from 0.1 M ammonium acetate, pH 7.0 to 0.5 M ammonium acetate, pH 7.0.

Example 3

Natriuretic factor assays were carried out in non-diuretic rats as follows:

Male Sprague-Dawley rats (wt. range 250-372 g) were anaesthetized (Inactin®, 10 mg/100 g body wt. i.p.) and prepared for bioassay. Arterial blood pressure and heart rate were measured through a femoral artery cannula. A bladder catheter allowed quantitative collection of urine, and a femoral vein cannula was used for maintenance infusion and injection of test material. On completion of surgery a priming dose of Ringer's solution (1.2 ml) containing $^3$H inulin (4.0 $\mu$Ci/ml) was administered over 20 min, followed by constant infusion of the same solution throughout the experiment at 1.2 ml/hr. The animals were let to stabilize for a further 20 min. During the following 20 min urine was quantitatively collected (control period) after which injection of 0.2 ml of the test sample was made over approximately 5 s. Urine was then collected for the next 20 min (test period). A photoelectric drop counter was positioned between the bladder catheter and the collection tube to qualitatively assess the diuretic response. Urine sodium and potassium concentrations were measured by flame photometry, chloride by electrometric titration and urine volumes by weighing. Protein estimation was made by measuring absorbance at 280 nm (10 mm path) assuming IAU - 1 mg protein/ml. Statistical comparisons were carried out using unpaired student's t-test.

Results

Pooled active freeze-dried fractions, obtained after chromatographic desalting in Bio-Gel® P-2 (Figure 1) or after fractionation in Sephadex® G-75 (Figure 2), and injected dissolved in PBS, induced a typical response in assay rats (Figure 3). In terms of urine output, this response was characterized by rapid onset (1-2 min) and decay (10-15 min). By the end of the test period - i.e. 20 min after injection - urine output was essentially the same as that observed during the control period. Doses approaching maximal response had the effect of gradually decreasing arterial blood pressure so that the blood pressure values observed at the end of the assay were 10-30 mm Hg lower than values observed prior to the injection of the extracts.

Both diuretic and natriuretic responses were dose dependent (Figure 4). Maximal response obtained after injection of 18 $\mu$g of partially purified factor corresponded to a 30-40 fold and 10-15 fold increase in total sodium excretion and urine volume respectively.

Incubation of partially purified natriuretic factor with protease is illustrated in Table I overleaf, and shows completely abolished activity while samples incubated with inactivated enzyme had activities comparable to that of samples incubated with buffer alone. Protease digestion was carried out by incubating approximately 50 $\mu$g of partially purified natriuretic factor with 0.1 U of protease for 4 h at 37 ° C in a total volume of 1.1 ml of 0.05M TRIS buffer, pH 7.6 (Group B). Controls were incubated with either boiled protease (Group C) or with buffer alone (Group A). (All values ± S.E.M.)

TABLE I

| | Group | Urine Volume µL/Min/G Kidney | Total Excretions (nEq/min/g kidney) $Na^+$ | $K^+$ | $Cl^-$ |
|---|---|---|---|---|---|
| Bioassay | A (N=6) | 2.39±0.49 | 117±27 | 643±137 | 573±158 |
| Control | B (N=4) | 2.03±0.84 | 68±6 | 316±52 | 151±46 |
| Period | C (N=4) | 2.14±0.73 | 102±39 | 578±221 | 819±250 |
| Bioassay | A (N.F.) | 13.20±3.24* | 1074±208* | 1902±513† | 2850±779* |
| Test | B (N.F. + Enz.) | 2.86±1.09† | 71±12† | 463±181† | 383±235† |
| Period | C (N.F. + Boiled Enz.) | 14.68±2.36** | 1434±190** | 1460±96* | 2549±343* |

*, **: Significant differences ($p<0.05$, $p<0.01$)

†: No significant differences from control values

Natriuretic factor distribution during chromatography in Sephadex® G-75 (Figure 2) was consistently multimodal although most of the activity was recovered in peaks corresponding to molecular weights of less than 6,000 Daltons. Further purification of this fraction was accomplished by ion exchange chromatography in CM Bio-Gel® (Figure 5) after which natriuretic activity was recovered in a single, though not symmetrical, peak.

The fact that natriuretic activity is recovered after desalting in a Bio-Gel® P-2 column (normal

fractionation range: 100-1,800 Daltons) is of considerable interest because it shows that natriuretic factor is distinct from inorganic salts and low molecular weight organic compounds which are likely extracted by acetic acid and which may be expected to be cardioactive and/or affect kidney function.

The significance of multimodal distribution of natriuretic factor after chromatography in Sephadex® G-75 is not clear. Protein interaction, possibly including polymerization, appears as the most likely explanation.

Protease sensitivity as well as the general behaviour of natriuretic factor suggest that it is a polypeptide.

The second technique for extraction of ANF follows a general outline for the isolation and purification of pituitary peptides described by Bennett et al "Purification of the Two Major Forms of Rat Pituitary Corticotrophin Using Only Reverse-Phase Liquid Chromatography" Biochemistry, 20: 4530-4558, (1981). Heart tissues are homogenized in an aqueous extractant mixture typically containing 1.0 M acetic acid, 1% sodium chloride and 1.0 M hydrochloric acid. Other organic acids, mineral acids or salts may, of course, also be used. The extracts are then treated with octadecylsilyl silica in a batch procedure and ANF is further purified using reverse phase high performance liquid chromatography.

Example 4

Rat atria, freshly dissected and frozen, were obtained commercially. The frozen tissue was finely ground and an acetone powder prepared by repeated extraction with acetone and hexane. Up to 700 mg of the dried powder was extracted three times with 20, 10 and 10 mL respectively of an aqueous extractant consisting of 1.0 M acetic acid, 1.0 M hydrochloric acid and 1% sodium chloride. Extraction was carried out on ice using a ground-glass homogenizer. The extracts so obtained were centrifuged and the supernatant was passed through two octadecylsilyl silica (ODS-silica) cartridges $C_{18}$ Sep-Pak®, Waters Associates). Before use, the cartridges were wetted with 5 ml of 80% acetonitrile (ACN)/water containing 0.1% trifluoroacetic acid (TFA) and rinsed with 5 ml of 0.1% TFA. After passing the samples, the cartridges were rinsed with 20 ml of 0.1% TFA. Compounds bound to the cartridges (including atrial natriuretic factor) were eluted by passing 3 ml of 80% ACN/0.1% TFA. The eluate from each cartridge was diluted to 18 mL with 0.1% TFA and pumped through the "aqueous" pump of a high performance liquid chromatograph for binding to a $\mu$-Bondapak® $C_{18}$ chromatographic column (Waters Associates) previously equilibrated with 12% ACN/0.1% TFA. The column was then eluted using a gradient of 20 to 40% ACN. Two active fractions are recovered. These fractions were further purified by separate rechromatography in the same system this time using ACN gradients containing 0.1% heptafluorobutyric acid (HFBA). Active fractions from HFBA gradients were purified once more by another gradient chromatography using TFA-containing ACN gradients. Injection of test samples of the extract thus produced into non-diuretic rats according to the procedure outlined in Example 3 resulted in similar results to those described in Example 3.

Example 5

Atria were obtained from adult male Sprague-Dawley rats. The tissues were kept frozen (-70 °C) from 24 to 72 h before being pulverized in a household-type coffee grinder previously cooled by grinding dry ice chips. The frozen tissue powder was then poured into a beaker containing 10 volumes of an ice-cold aqueous extractant composed of 1.0 M acetic acid, 1.0 N HCL and 1% NaCl and homogenized using a Polytron fitted with a PT35 probe operated for 60 s at 50% power. The homogenate was stirred for 1 h in the cold before centrifuging. The pellets thus obtained were re-extracted following an identical protocol but using 5 volumes of the extractant. The supernatants from the two extractions were combined and 40 ml aliquots were passed five times through Sep-Pak® cartridges (Waters) which were then washed with 20 ml of 0.1% trifluoracetic acid (TFA) and eluted with 3 ml of 80% acetonitrile ($CH_3CN$) in 0.1% TFA. The eluates were then diluted with 0.1% TFA in a proportion of 15 ml of acid to 3 ml of cartridge eluate. The diluted eluate was pumped into a HPLC column ($\mu$-Bondapak® $C_{18}$, 7.8 x 300 mm) through the "B" (aqueous) pump of a series 2/2 Perkin-Elmer liquid chromatograph. The column had previously be equilibrated with 12% $CH_3CN$ in 0.1% TFA and gradient elution was carried out over 75 min at 1.5 ml/min with a linear gradient of 20 - 50% $CH_3CN$ in 0.1% TFA. Column effluent was monitored at 278 nm (LC55 Perkin-Elmer detector) and collected in 2 min fractions. Aliquots of the fractions were freeze-dried and re-suspended in phosphate-buffered saline (PBS) for natriuretic factor assay. Figure 6 shows the elution profile of extracts obtained from 200 atria after RP-HPLC. The extract obtained from 200 rat atria was pumped directly into the chromatographic column ($\mu$-Bondapak® $C_{18}$, 7.8 x 300 mm) and eluted over 75 min with a gradient of 20-50% $CH_3CN$ in 0.1% TFA. Two min fractions were collected. Aliquots of these fractions were freeze-dried and re-suspended in PBS for assay of natriuretic activity. Activity was found distributed in four discrete regions of the chromatogram. The fractions comprising region I were pooled, diluted 1:1 with aqueous

0.13% heptafluorobutyric acid (HFBA) and pumped back into the column which had been equilibrated with 12% $CH_3CN$ in 0.13% HFBA. Elution of the column was carried out over 40 min with a gradient of 28 - 44% $CH_3CN$ in 0.13% HFBA. Activity was recovered in fractions eluting between 31 and 34 min (Fig. 7). These fractions were diluted 1:1 with 0.1% aqueous TFA and re-chromatographed using a 3.9 x 300 mm $\mu$-Bondapak® $C_{18}$ column eluted over 20 min with a gradient of 20 - 36% $CH_3CN$ in 0.1% TFA. As shown in Figure 8, a sharp, symmetrical peak was now obtained which was apparently homogeneous at both 278 nm (Figure 8a) and 215 nm (Figure 8b).

The product obtained at this point is hereinafter referred to as "cardionatrin I". Total yield of cardionatrin I varied from 12 - 20 nmol per 1,000 atria. The material obtained from 200 rat atria was used to obtain a molecular weight estimate using area-sodium dodecylsulfate-polyacrylamide gel electrophoresis (SDS-PAGE).

Further characterization studies on "cardionatrin I" have been carried out using preparations of cardionatrin I derived from 1,000 rat atria, as follows: amino acid analysis after hydrolysis in 6 N HCL both with or without prior performic acid oxidation; amino acid analysis after hydrolysis in 4 N methanesulfonic acid; spectrophotometric determination of tryptophan.

The molecular weight of cardionatrin I was estimated by SDS-PAGE. Cardionatrin I (I) obtained from a 200-rat atrial extract, was analyzed by urea SDS-PAGE together with molecular weight standards. The standards were cyanogen bromide fragments of sperm whale myoglobin (MF) and commercially obtained glucagon (G). Plot of molecular weight versus migrated distance (Fig. 9) gives an estimate of 5,150 for the molecular weight of cardionatrin I (I).

Amino acid analysis (Table II)

Table II.

| Amino Acid Composition of Cardionatrin I | |
|---|---|
| Amino Acid | No. of Residues |
| Asp | 3.7 (4) |
| Thr | 1.3 (1) |
| Ser | 6.4 (6) |
| Glx | 4.8 (5) |
| Pro | 1.9 (2) |
| Gly | 7.3 (7) |
| Ala | 3.3 (3) |
| Cys | 0.9 (1) |
| Val | 1.0 (1) |
| Met | 0.8 (1) |
| Leu | 3.2 (3) |
| Tyr | 1.1 (1) |
| Phe | 1.8 (2) |
| Lys | 2.8 (3) |
| His | 1.1 (1) |
| Trp | 0.0 (0) |
| Arg | 5.7 (6) |
| Total no. of residues | 47 |

revealed that the peptide consisted of 47 residues one of which was cystine indicating that cardionatrin I contains a disulfide bond. No trytophan residue was detected by amino acid analysis following hydrolysis of the peptide in 4 N methanesulfonic acid. The UV spectrum of the peptide also did not show the shoulder at 288 nm characteristic of tryptophan containing peptides (Fig. 10). The spectrum was obtained using a Cary 210 spectrophotometer with a 10 mm light path and equipped with automatic baseline adjust. For the spectrum shown, 10 nmol of cardionatrin I were dissolved in 1.0 mL of 0.1 N HCL. The molecular weight of cardionatrin I estimated from the amino acid composition was 5273 which is in close agreement with that estimated by SDS-PAGE gel electrophoresis.

Injection of 0.5 nmol of purified cardionatrin I induced a characteristic diuretic response of rapid onset

and decay in the non-diuretic bioassay rat, resulting in a two-fold increase in urine output and a four-fold increase in sodium excretion. From previous dose-response studies using partially purified preparations, maximal bioassay response for cardionatrin I may be expected to be in the 1 to 2 nmol range.

Experiments to determine the sequence of the amino acids in the peptide described above, or at least that portion of the cardionatrin I which exhibits biological activity, i.e. diuretic properties, have been conducted using a Beckman 890C automatic sequencer. Edman degradation followed by analysis of phenylthiohydantoin amino-acids by high pressure liquid chromatography resulted in a derived sequence as follows:

Serine - Leucine - Arginine - Arginine - Serine -

Serine - Cysteine - Phenylalanine - Glycine - Glycine -

S Arginine - Isoleucine - Aspartic acid - Arginine -

S Isoleucine - Glycine - Alanine - Glutamine -

Serine - Glycine - Leucine - Glycine - Cysteine -

Asparagine - Serine - Phenylalanine - (Arginine) -

Tyrosine.

A similar methodology was employed using an Applied Biosystems gas phase sequencer in order to confirm the same linear sequence. The molecular weight of this peptide is about 3,500.

It will, of course, be appreciated that the presence of the relatively short disulfide bond between the relatively widely spaced cysteine groups indicates that the structure is not linear but is folded in such a manner that the cysteine groups are sufficiently close to each other to be bridged by the disulfide bond. It will be further appreciated that following establishment of the amino acid linear sequence synthesis of the peptide of the present invention may be readily effected by standard biochemical techniques or by recombinant DNA technology.

The ability of either crude atrial extracts or partially purified atrial natriuretic factor to induce a rapid and potent diuretic and natriuretic response in bioassay animals has been noted above. In addition, recent tissue fractionation studies, have shown that this activity appears largely stored in the secretory-like specific atrial granules. These granules share histochemical and tinctorial properties with granules known to store polypeptide hormones. Histochemical data suggest the presence of sulphur-containing amino acids in atrial granules and cardionatrin I contains a cystine residue and a methionine residue. However, the histochemical studies also indicate the presence of indole in atrial granules, but the amino acid composition of cardionatrin I showed that no tryptophan was present. The reasons for this apparent discrepancy are not clear at present but could be related to the specificity of the histochemical technique or the presence of other indole containing species within atrial granules. Clarification of this may be expected with the characterization of the remaining peptides with natriuretic activity found after RP-HPLC of atrial homogenates.

The ANF is clearly a highly useful pharmaceutical composition for diuretic and natriuretic purposes and is also believed to be of great potential in reaching a better understanding of the in vivo control of water and electrolyte balance. Should future investigations show that peptides such as cardionatrin I are released from the atria to modify kidney function then this would have a major impact on the understanding of disturbances of water and electrolyte balance which occur in such clinical entities as essential hypertension and chronic congestive heart failure.

## Claims

1. A peptide composition having diuretic and natriuretic properties, characterized by an amino acid sequence comprising:

Serine - Leucine - Arginine - Arginine - Serine -
Serine - Cysteine - Phenylalanine - Glycine -
S Glycine - Arginine - Isoleucine - Aspartic acid -
Arginine - Isoleucine - Glycine - Alanine -
Glutamine - Serine - Glycine - Leucine -
S Glycine - Cysteine - Asparagine - Serine -
Phenylalanine-(Arginine) - Tyrosine.

2. A polypeptide containing the sequence
- Cysteine - Asparagine - Serine - phenylalanine - Arginine - Tyrosine at the C-terminal thereof, and having a disulfide bond between the said cysteine residue and another cysteine residue in the polypeptide.

3. A substantially pure polypeptide having hypotensive, diuretic and natriuretic properties, said diuretic and natriuretic properties enabling the polypeptide to increase the normal diuretic and natriuretic extretions, causing at least a two fold increase in urine output and four fold increase in sodium excretion, on injection of 0.5 nmol of said polypeptide into a non-diuretic rat, said polypeptide being free of tryptophan residues and containing cysteine residues which are bridged by a disulfide double bond.

**Revendications**

1. Composition de peptides ayant des propriétés diurétiques et natriurétiques, caractérisée par une séquence d'acides aminés comprenant :

Sérine - Leucine - Arginine - Arginine- Sérine -
Sérine - Cystéine - Phénylalanine - Glycine -
S Glycine - Arginine - Isoleucine - Acide aspartique-
Arginine - Isoleucine - Glycine - Alanine -
Glutamine - Sérine - Glycine - Leucine-
S Glycine - Cystéine - Asparagine - Sérine -
Phénylalanine-(Arginine) - Tyrosine.

2. Polypeptide contenant la séquence
- Cystéine - Asparagine - Sérine - Phénylalanine - Arginine - Tyrosine à son extrémité C, et ayant une liaison disulfure entre le résidu de cystéine et un autre résidu de cystéine du polypeptide.

3. Polypeptide sensiblement pur, ayant des propriétés hypotensives, diurétiques et natriurétiques, ces propriétés diurétiques et natriurétiques permettant au polypeptide d'augmenter les excrétions diurétiques et natriurétiques normales, en provoquant au moins un doublement du débit urinaire et au moins un quadruplement de l'excrétion du sodium, par injection de 0,5 nmole du polypeptide à un rat non diurétique, le polypeptide étant exempt de résidu tryptophan et contenant des résidus cystéine qui sont pontés par une double liaison disulfure.

**Patentansprüche**

1. Peptidzusammensetzung mit diuretischen und natriuretischen Eigenschaften, *gekennzeichnet* durch eine Aminosäuresequenz mit:

Serin - Leucin - Arginin - Arginin - Serin -
Serin - Cystein - Phenylalanin - Glycin -
Glycin - Arginin - Isoleucin - Asparaginsäure - S
Arginin - Isoleucin - Glycin - Alanin -
Glutamin - Serin - Glycin - Leucin - S
Glycin - Cystein - Asparagin - Serin -
Phenylalanin - (Arginin) - Tyrosin

2. Polypeptid, das C-terminal die Sequenz - Cystein - Asparagin - Serin - Phenylalanin - Arginin - Tyrosin aufweist und eine Disulfidbindung zwischen dem Cysteinrest und einem anderen Cysteinrest im Polypeptid besitzt.

3. Im wesentlichen reines Polypeptid mit hypotensiven, diuretischen und natriuretischen Eigenschaften, wobei die diuretischen und natriuretischen Eigenschaften das Polypeptid in die Lage versetzen, die normalen diuretischen und natriuretischen Ausscheidungen zu erhöhen, so daß es zu einer mindestens zweifachen Erhöhung des Urinausstoßes und vierfachen Erhöhung der Natriumausscheidung infolge der Injektion von 0,5 nmol des Polypeptids in eine nicht-diuretische Ratte kommt, wobei das Polypeptid frei von Tryptophanresten ist und Cysteinreste enthält, die durch eine Disulfidgruppe brückenartig verbunden sind.

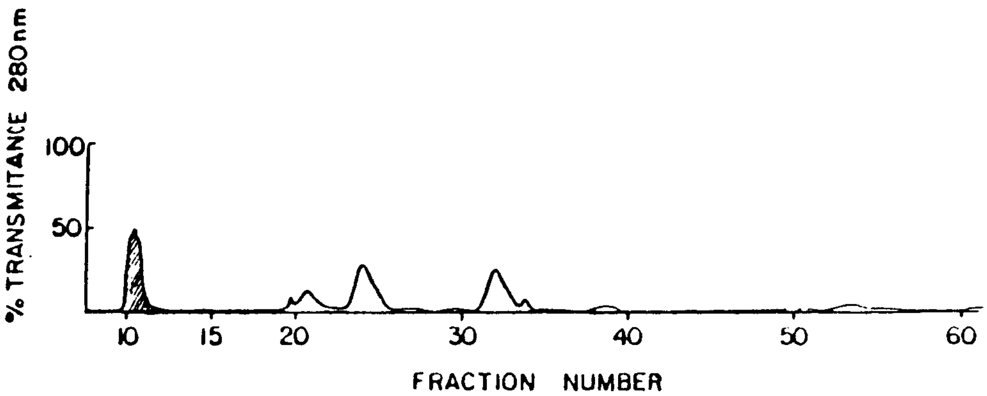
% TRANSMITANCE 280nm
100
50
10
15
20
30
40
50
60
FRACTION NUMBER

FIG. 1

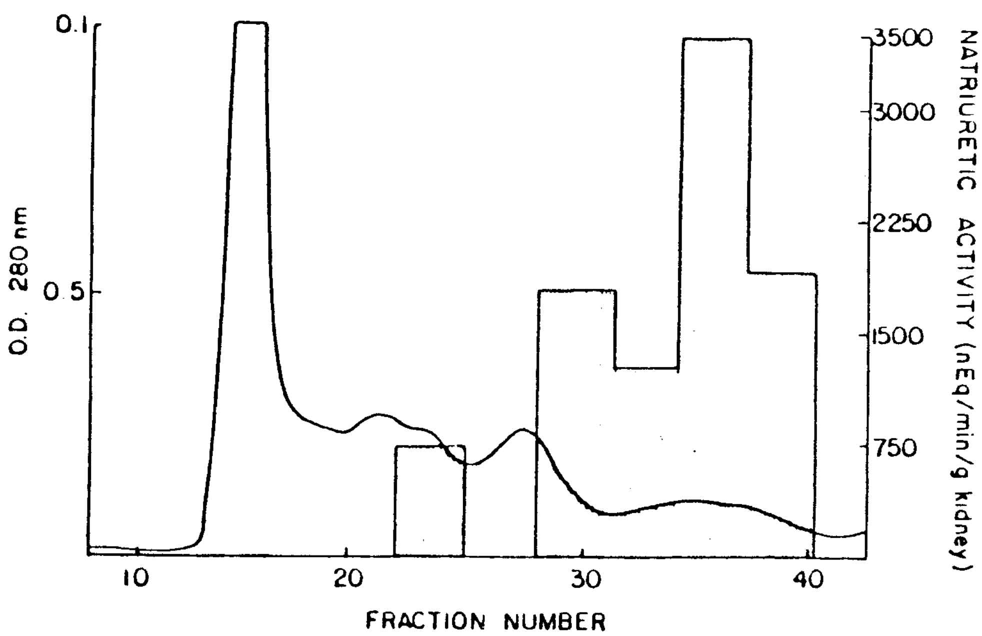
0.1
0.5
O.D. 280nm
3500
3000
2250
1500
750
NATRIURETIC ACTIVITY (nEq/min/g kidney)
10
20
30
40
FRACTION NUMBER

FIG. 2

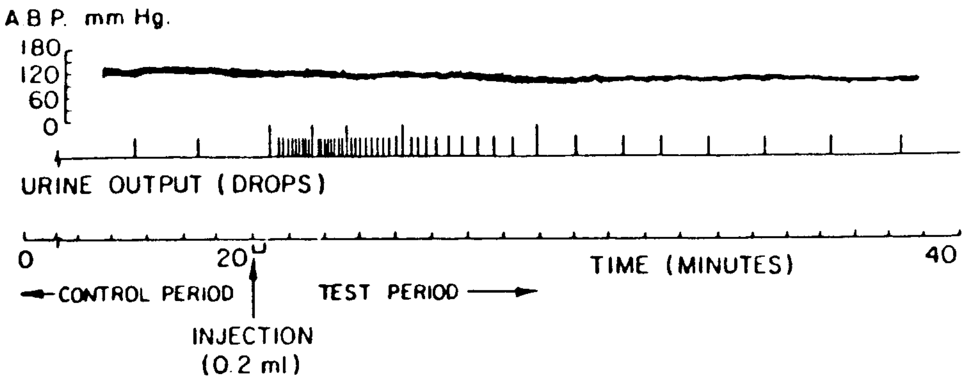
A.B.P. mm Hg.
180
120
60
0
URINE OUTPUT (DROPS)
0
20
TIME (MINUTES)
40
CONTROL PERIOD
TEST PERIOD
INJECTION
(0.2 ml)

FIG. 3

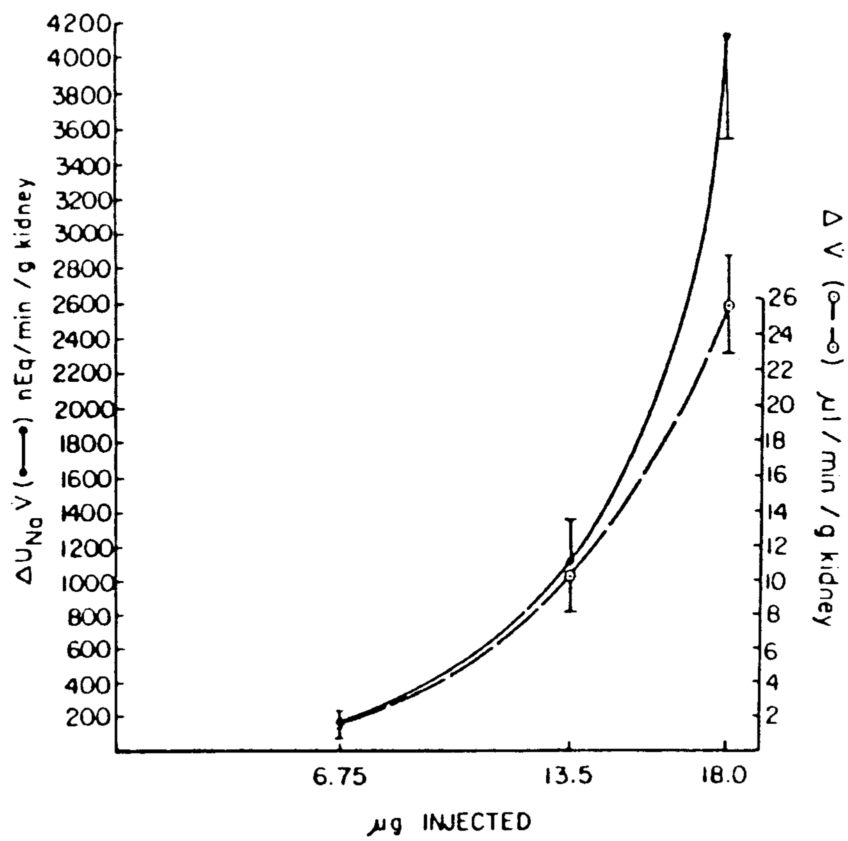
4200
4000
3800
3600
3400
3200
3000
2800
2600
2400
2200
2000
1800
1600
1400
1200
1000
800
600
400
200
ΔU_Na V̇ (•—•) nEq/min/g kidney
26
24
22
20
18
16
14
12
10
8
6
4
2
ΔV̇ (○—○) µl/min/g kidney
6.75
13.5
18.0
µg INJECTED

FIG. 4

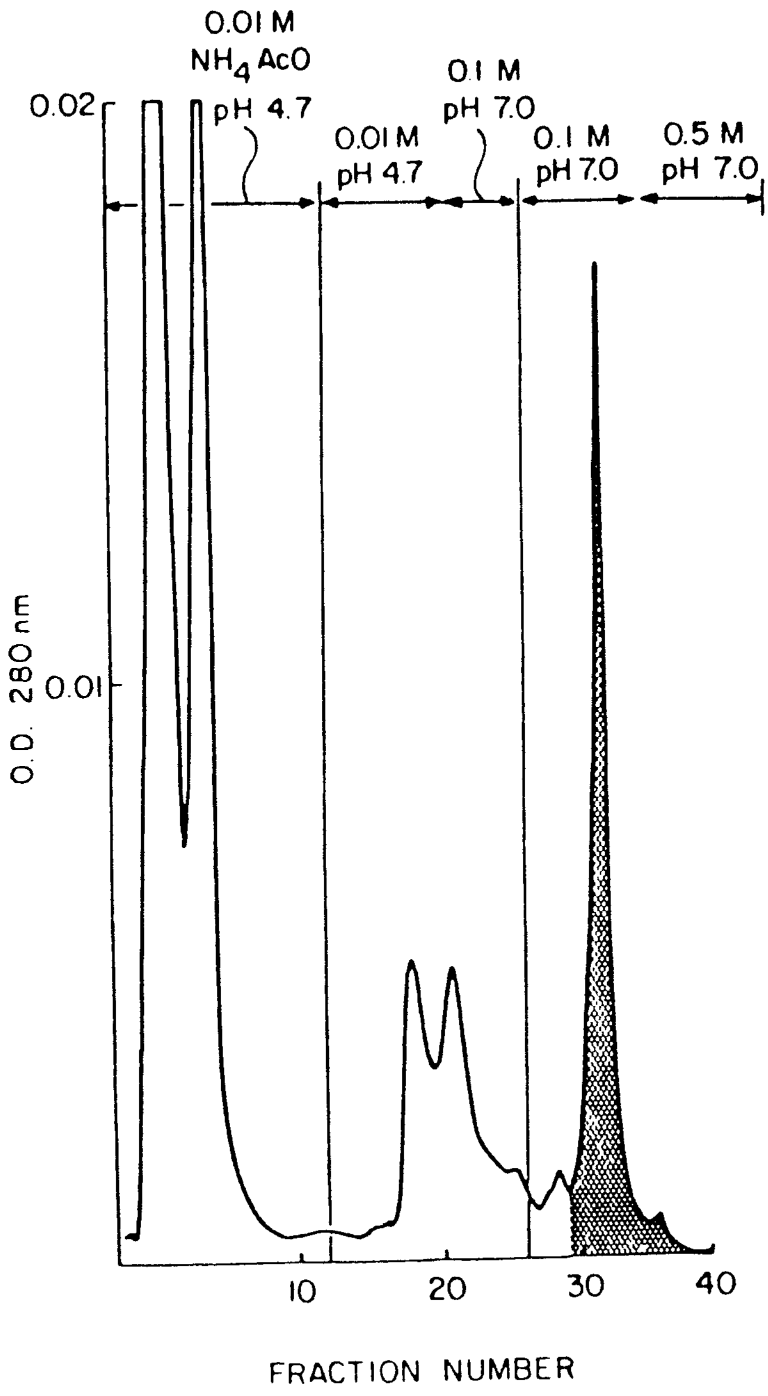
0.01 M
NH4 AcO
pH 4.7
0.01 M
pH 4.7
0.1 M
pH 7.0
0.1 M
pH 7.0
0.5 M
pH 7.0
0.02
0.01
O.D. 280 nm
10
20
30
40
FRACTION NUMBER

FIG. 5

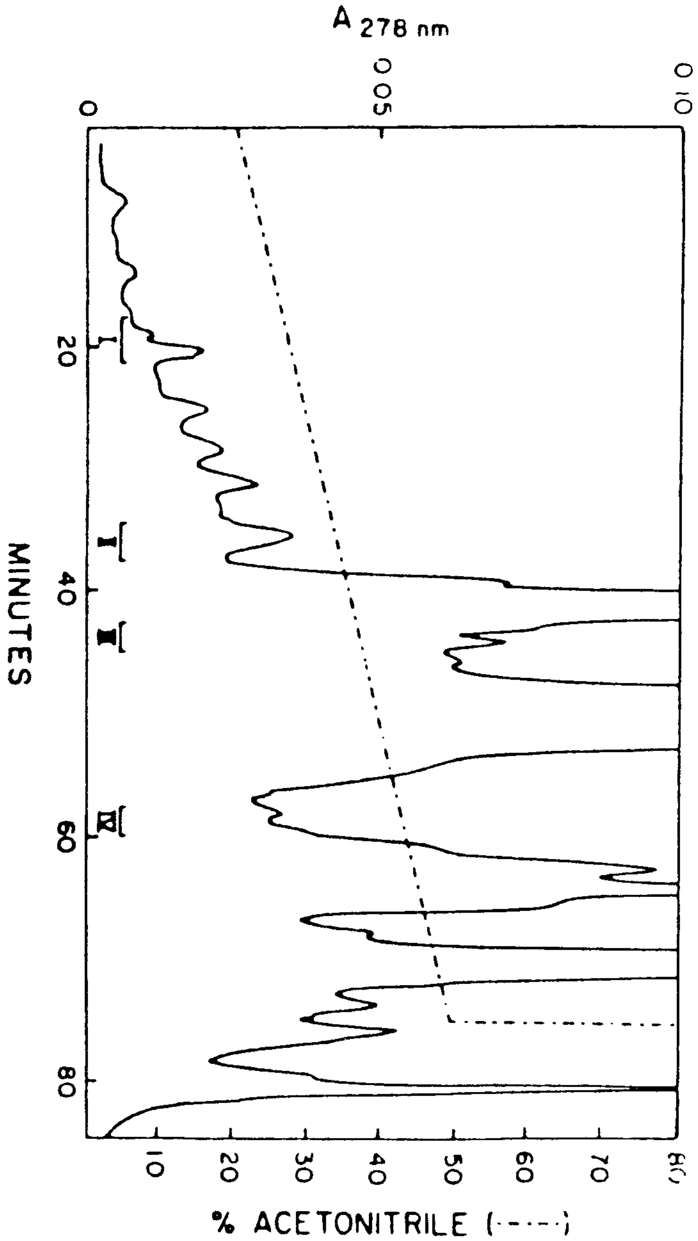
A 278 nm
0
0.05
0.10
MINUTES
20
40
60
80
I
II
III
IV
10
20
30
40
50
60
70
80
% ACETONITRILE (·-·-·)

FIG. 6

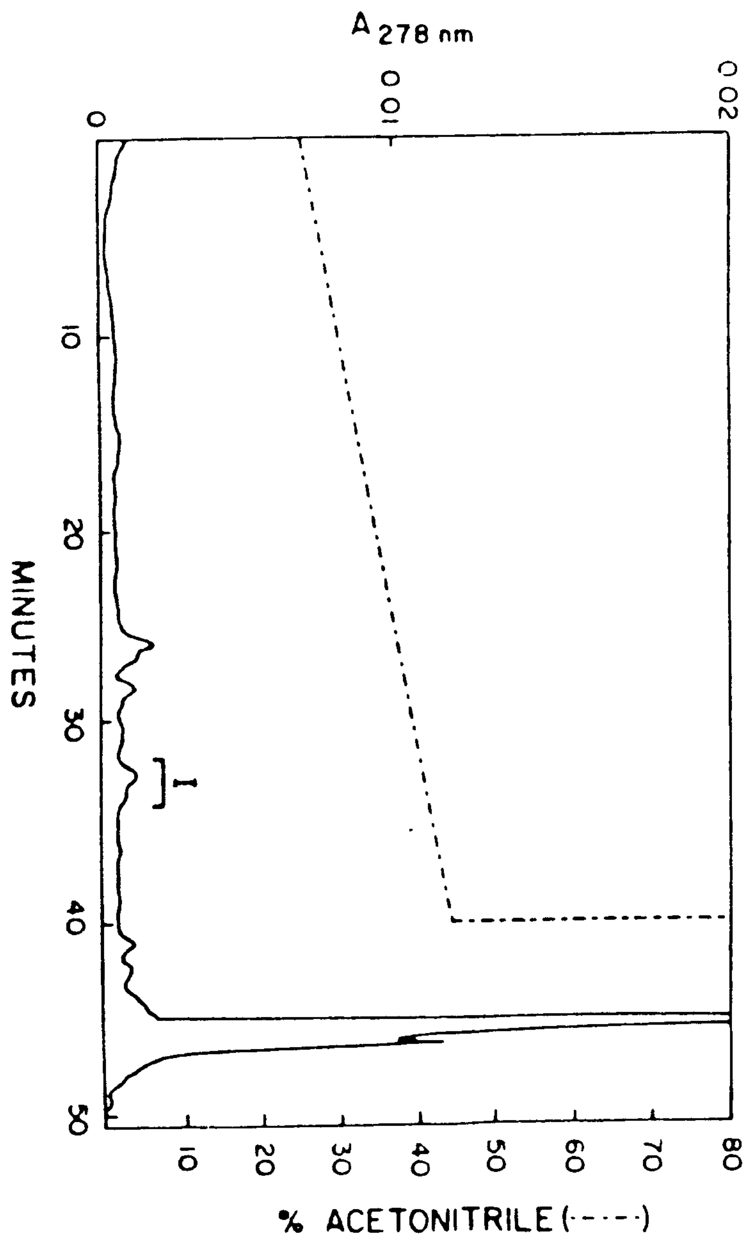

A 278 nm
0
0.01
0.02
MINUTES
10
20
30
40
50
I
10
20
30
40
50
60
70
80
% ACETONITRILE (-·-·-)


FIG. 7

FIG. 8a

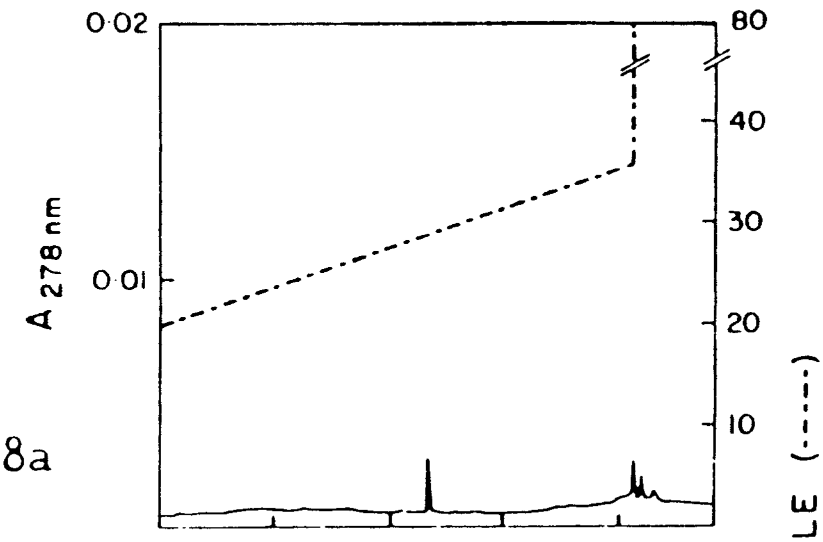
0·02
0·01
A 278 nm
80
40
30
20
10
% ACETONITRILE (-·-·-)

FIG. 8b

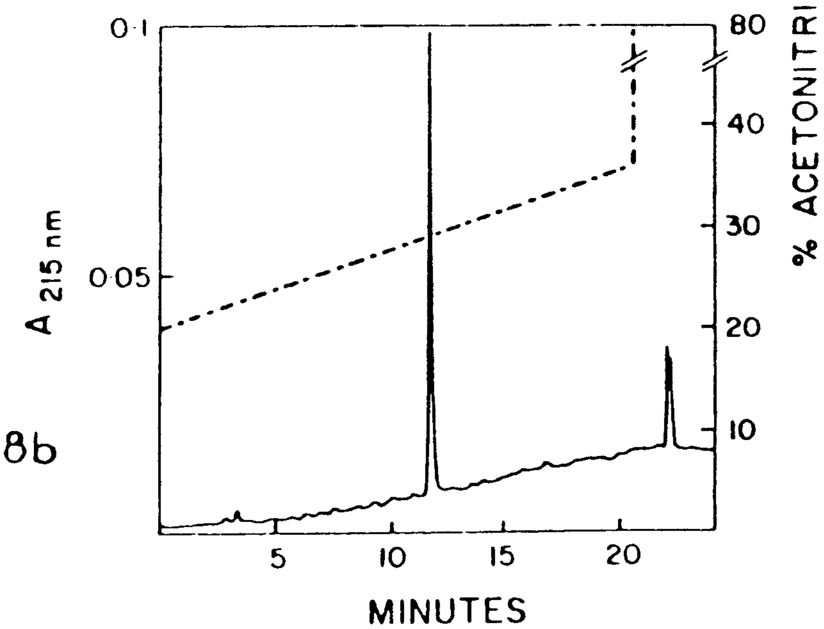
0·1
0·05
A 215 nm
80
40
30
20
10
5
10
15
20
MINUTES

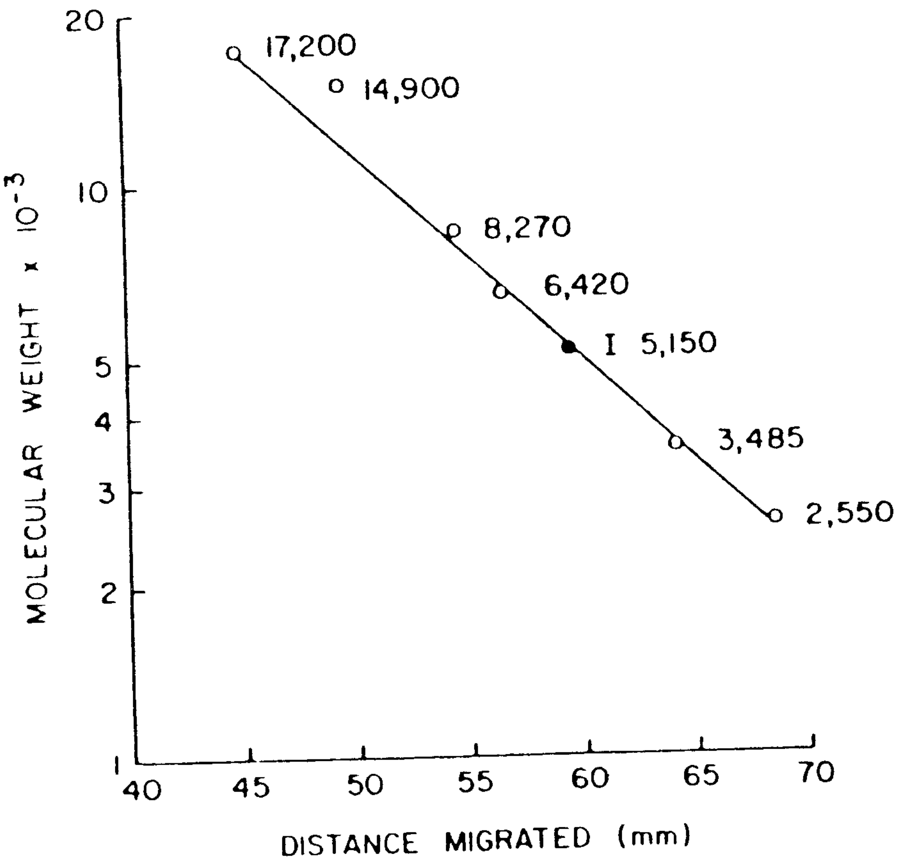
20
10
5
4
3
2
1
MOLECULAR WEIGHT x 10-3
17,200
14,900
8,270
6,420
I 5,150
3,485
2,550
40
45
50
55
60
65
70
DISTANCE MIGRATED (mm)

FIG. 9

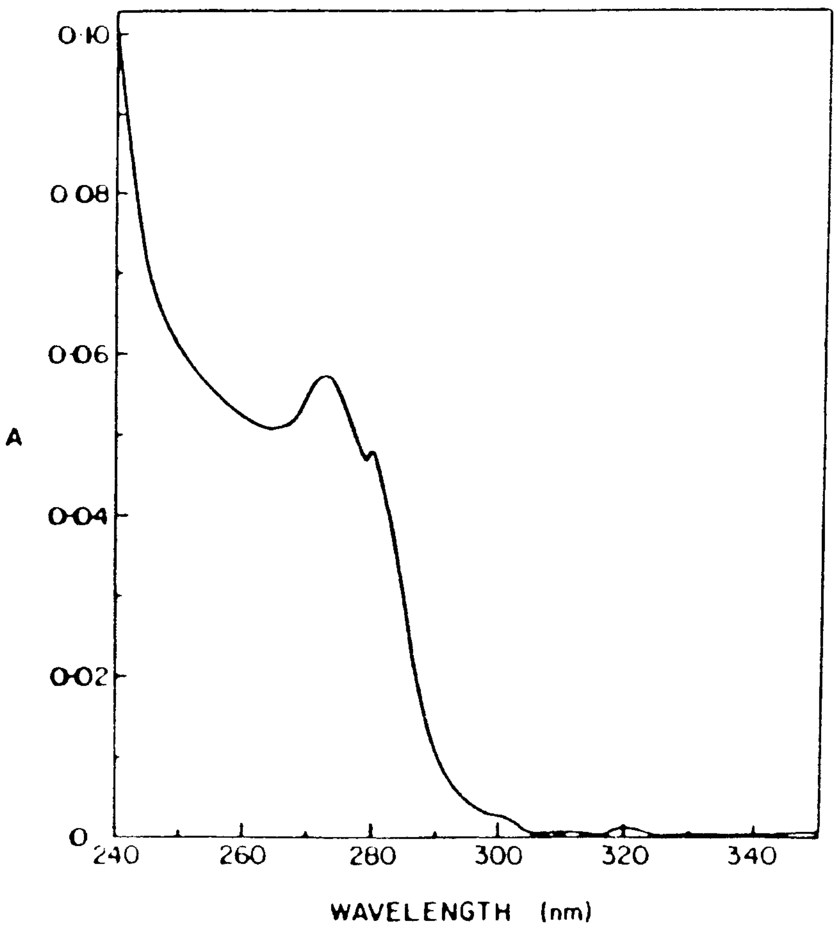


FIG. 10